## Europäisches Patentamt

## European Patent Office

## Office européen des brevets

(19)

(11) Publication number: **0 009 289**
**B1**

(12)

# EUROPEAN PATENT SPECIFICATION

(45) Date of publication of patent specification: **02.06.82**

(21) Application number: **79200516.7**

(22) Date of filing: **17.09.79**

(51) Int. Cl.³: **C 07 D 211/02,
C 07 D 213/08,
C 07 D 215/04, C 01 B 3/00**

(54) **Process for the preparation of C-substituted pyridines and/or hydrogenated C-substituted pyridines.**

(30) Priority: **20.09.78 NL 7809552**

(43) Date of publication of application:
**02.04.80 Bulletin 80/7**

(45) Publication of the grant of the patent:
**02.06.82 Bulletin 82/22**

(84) Designated Contracting States:
**BE CH DE FR GB IT NL SE**

(56) References cited:
**GB - A - 1 062 900
NL - A - 70 04993
US - A - 3 695 841**

(73) Proprietor: **STAMICARBON B.V.
Postbus 10
NL-6160 MC Geleen (NL)**

(72) Inventor: **Verheijen, Egidius Johannes Maria
Havenweg 74
NL-6121 CN Born (NL)**
Inventor: **Geersheuvels, Charles Hendricus
van Ostadestraat 76
NL-6165 XM Geleen (NL)**

(74) Representative: **Pinckaers, August René et al,
OCTROOIBUREAU DSM Postbus 9
NL-6160 MA Geleen (NL)**

Courier Press, Leamington Spa, England.

# Process for the preparation of C-substituted pyridines and/or hydrogenated C-substituted pyridines

The invention relates to a process for the preparation of pyridines and/or hydrogenated pyridines that have been substituted with a hydrocarbon group at one or more C-atoms by passing a $\gamma$-cyanoketone in the gaseous phase and in the presence of hydrogen over a catalyst containing a metal or a compound of a metal from the group consisting of copper, silver, gold, iron, nickel, cobalt, ruthenium, rhodium, palladium, osmium, iridium, and platinum.

A process of this type, in which compounds can be obtained that can be used in the preparation of pharmaceutical products and insecticides, has been described in British Patent Specifications 1,304,155 and 1,425,698. The gaseous reaction mixture obtained in this known process contains a rather large amount of hydrogen that can be re-used after being separated from the reaction mixture.

However, when experiments were performed in which said hydrogen, which usually contains 5—20% of other gases, was recycled, it was found that the selectivity and the activity of the catalyst used dropped much more rapidly than without such recirculation of hydrogen.

A method has now been found in which the gas containing hydrogen that is separated from the resulting reaction mixture can be recycled without any adverse effect on the selectivity and activity of the catalyst.

The process according to the invention for the preparation of pyridines and/or hydrogenated pyridines that have been substituted with a hydrocarbon group at one or more C-atoms by passing a $\gamma$-cyanoketone in the gaseous phase in the presence of hydrogen over a catalyst containing a metal of a compound of a metal from the group consisting of copper, silver, gold, iron, nickel, cobalt, ruthenium, rhodium, palladium, osmium, iridium and platinum, separating gas containing hydrogen from the resulting reaction mixture and recycling it is characterized in that the hydrogen-containing gas to be recycled is passed, at a temperature of 450—650°C, over a catalyst containing a metal from the group consisting of nickel and cobalt and in that the hydrogen-containing gas is passed over the catalyst with a space velocity of 0.25—20 litres (N.T.P.) per gram of catalyst and per hour.

In the process according to the invention, the catalysts containing nickel and cobalt that are commercially available may be used for the treatment of the hydrogen-containing gas to be recycled. These catalysts usually contain a supporting material, such as, e.g., alumina, silica, magnesia and carbon.

It is known that hydrogen obtained by dehydrogenation of hydrocarbons can be purified by passing said hydrogen over a catalyst comprising nickel on aluminium-oxide and/or magnesiumoxide at elevated temperature preferably of from 200° to 350°C (U.S.A. specification 3,695,841). The hydrogen to be recycled in the process according to the invention however, is not hydrogen obtained in a dehydrogenation reaction but hydrogen which has been used in the cyclization of a cyanoketone and which contains other impurities. The said preferred temperatures of from 200°—350°C are not employed in the treatment of the hydrogen to be recycled in the process according to the invention.

It is also known (British specification 1,062,900) that in the hydrogenation of a pyridine the catalyst life can be extended by passing the pyridine together with hydrogen through a primary reactor before feeding it to the main reactor, the primary reactor being filled with used but hydrogenation-inactive catalyst of the same kind and from the same hydrogenation process as the subsequent hydrogenation. In the process according to the invention, the hydrogen to be recycled is passed over the concerning catalyst in the absence of the cyanoketone while the concerning catalyst is not a catalyst which has been used in the cyclization reaction of the cyanoketone.

The pressure at which the hydrogen-containing gas to be recycled is passed over the catalyst is not critical and, consequently, atmospheric pressure is used by preference.

Just as the known process, the process according to the invention may use various $\gamma$-cyanoketones as the starting compound, such as, e.g. 5-oxohexane nitrile, 5-oxoheptane nitrile, 4 - methyl - 5 - oxohexane nitrile, 2 - ($\beta$ - cyanoethyl) cyclohexanone, 4 - phenyl - 5 - oxohexane nitrile, and 4 - methyl - 5 - oxoheptane nitrile.

The process according to the invention is very suitable for the preparation of 2 - methyl - pyridine, 2,3-lutidine and quinoline from the respective starting products 5-oxohexane nitrile, 4 - methyl - 5 - oxohexane nitrile and 2 - ($\beta$ - cyanoethyl) - cyclohexanone.

When the process according to the invention is effected, it may happen that the original hydrogen is not fully pure and that impurities are formed in the conversion of the cyanoketone. If so, too high a concentration of impurities can be avoided by venting part of the hydrogen to be recycled.

The process according to the invention will be further elucidated in the following examples.

Examples I—V

A gaseous mixture of 5-oxohexane nitrile and hydrogen obtained by evaporation of liquid 5-oxohexane nitrile and mixing the resulting vapour with hydrogen was passed over the catalyst bed (50 grams of catalyst consisting of palladium on alumina with 0,5% by weight of Pd) in a tubular reactor of 25 millimetres in diameter and 500 millimetres in length equipped with a heating jacket. The temperature of the catalyst bed was kept at 240°C. The space velocity of the nitrile was 0.15 gram per gram of catalyst and per hour and that of the hydrogen was 0.15 litre (N.T.P.) of hydrogen per gram of catalyst and per hour.

The resulting reaction mixture was passed through an ice-cooled collecting vessel in which the reaction product condensed. The noncondensed gaseous reaction mixture, which mainly consisted of hydrogen, was passed at elevated temperature (varied between 475 and 525°C) through a second tubular reactor (diameter 10 millimetres, length 250 millimetres) containing a bed of 2 grams of nickel catalyst (16.1% by weight of Ni on $SiO_2$, type G1-22 of BASF) and then recycled to the inlet of the reactor in which the 5-oxohexane nitrile was converted. The space velocity was varied between 0.75 and 7.5 litres (N.T.P.) of hydrogen per gram of nickel catalyst and per hour. After an operating period of 100 hours the amount of 5-oxohexane nitrile passed through and the amount of reaction product obtained was measured for 1 hour under constant conditions. The amount of 5-oxohexane nitrile passed through was determined by measuring the loss in weight of liquid 5-oxohexane nitrile.

The collected reaction product was analysed gas-chromatographically. The results of the determinations are compiled in the table below. This table also states the results of two comparative examples. Comparative examples A relates to an experiment in which no hydrogen was recycled to the reactor, while comparative example B relates to an experiment in which the hydrogen supplied to the reactor consisted of 90% of recycled hydrogen, which, however, was recycled directly without any treatment.

| example | temperature nickel cat. °C | space velocity 2nd reactor | conversion of nitrile % | selectivity 2-methyl pyridine % | selectivity 2-methyl piperidine % |
|---|---|---|---|---|---|
| I | 550 | 3.75 | 100 | 84.0 | 4.7 |
| II | 475 | 3.75 | 99.8 | 83.8 | 4.5 |
| III | 600 | 3.75 | 100 | 84.1 | 4.6 |
| IV | 550 | 7.50 | 99.9 | 83.7 | 4.7 |
| V | 550 | 0.75 | 100 | 84.0 | 4.6 |
| A | — | — | 100 | 84.2 | 4.6 |
| B | — | — | 94.8 | 75.1 | 4.7 |

Example VI

The experiment of example I was repeated under similar conditions, but with the use of another catalyst for the treatment of the hydrogen to be recycled, viz. the Co-0127 catalyst of Harshaw (cobalt on kieselguhr with a cobalt content of 39% by weight). After an operating period of 100 hours the conversion of the nitrile was 100%, the selectivity to 2-methyl pyridine 83.9% and that to 2-methyl piperidine 4.6%.

Example VII

Example I and comparative examples A and B were repeated under the same conditions, but at 230°C with 4 - methyl - 5 - oxohexane nitrile instead of 5-oxohexane nitrile.

The conversion was 99.3%, the selectivity to 2,3 - di - methyl pyridine 89,3% and that to 2,3-dimethyl piperidine 9.2%. In the comparative example without recirculation of hydrogen these values were 99.6, 89.7 and 8.6%, respectively, and in the comparative example with recirculation of the untreated hydrogen the values were 94.3, 79.3 and 9.3%.

Example VIII

Example I and the comparative examples A and B were repeated under the same conditions, but at 230°C with 5-oxo-heptane nitrile instead of 5-oxohexane nitrile.

The conversion was 99.2%, the selectivity to 2-ethylpyridine 80.3% and that to 2-ethyl piperidine 13.6%. In the comparative example without hydrogen recirculation these values were 99.7%, 80.4% and 13.3%, respectively, and in the comparative example with recirculation of untreated hydrogen the values were 94.5, 74.5 and 14.2%, respectively.

Example IX

2-$\beta$-cyanoethyl cyclohexanone was passed over 50 grams of the Pd catalyst in the way described in the example I. The temperature of the catalyst was kept at 210°C. The space velocity of the cyanoketone was 0.15 gram per gram of catalyst and per hour and that of the hydrogen 0.3 litre (N.T.P.) per gram of catalyst and per hour. The hydrogen to be recycled was passed over the same nickel catalyst as in example I at a temperature of 550°C with the same space velocity as in example I. For comparison, an experiment was effected without recirculation of hydrogen and an experiment with recirculation of untreated hydrogen, under similar conditions.

In the example, the comparative experiment without recirculation and the comparative experiment with said recirculation, the conversion was 100, 100, and 96.4%, respectively, the selectivity to quinoline was 1% in all three cases, the selectivity to decahydroquinoline 48, 48 and 44%, respectively, the selectivity to 1,2,3,4-tetrahydroquinoline 6, 6 and 7%, respectively, and the selectivity to 5,6,7,8-tetrahydroquinoline 41, 42 and 39%, respectively.

## Claim

Process for the preparation of pyridines and/or hydrogenated pyridines that have been substituted with a hydrocarbon group at one or more C-atoms by passing a $\gamma$-cyanoketone in the gaseous phase and in the presence of hydrogen over a catalyst containing a metal or a compound of a metal from the group consisting of copper, silver, gold, iron, nickel, cobalt, ruthenium, rhodium, palladium, osmium, iridium, and platinum, separating gas containing hydrogen from the resulting reaction mixture and recycling it, characterized in that the hydrogen-containing gas to be recycled is passed, at a temperature of 450—460°C, over a catalyst containing a metal from the group consisting of nickel and cobalt and in that the hydrogen-containing gas is passed over the catalyst with a space velocity of 0.25—20 litres (N.T.P.) per gram of catalyst and per hour.

## Patentanspruch

Verfahren zur Herstellung von Pyridinen und/oder hydrierten Pyridinen, die mit einer Kohlenwasserstoffgruppe an einem oder mehreren C-Atomen substituiert sind, durch überleiten eines $\gamma$-Cyanoketons in der Gasphase und in Gegenwart von Wasserstoff über einen Katalysator, der ein Metall oder ein Metallverbindung aus der Gruppe Kupfer, Silber, Gold, Eisen, Nickel, Kobalt, Ruthenium, Rhodium, Palladium, Osmium, Iridium und Platin enthält, Trennung des Wasserstoff enthaltenden Gases von de resultierenden Reaktionsgemisch und Rückführung dieses Gases, dadurch gekennzeichnet, dass das rüchzuführende Wasserstoff enthaltende Gas bei einer Temperatur von 450—650°C über einen Katalysator geleitet wird, der ein Metall aus der Gruppe Nickel und Kobalt enthält und in dem das Wasserstoff enthaltende Gas mit einer Raumgeschwindigkeit von 0,25—20 Litern (normaler Druck und Temperatur) pro Gramm des Katalysators und pro Stunde über den Katalysator geleitet wird.

## Revendication

Procédé de préparation de pyridines et/ou de pyridines hydrogénées qui, à un ou à plusieurs atomes de C, ont été substituées d'un groupe d'hydrocarbures, en conduisant une $\gamma$-cyanocétone en phase gazeuse et en présence d'hydrogène sur un catalyseur contenant un métal ou un composé de métal du groupe constitué de cuivre, argent, or, fer, nickel, cobalt, ruthénium, rhodium, palladium, osmium, iridium, et platine, en séparant le gaz contenant de l'hydrogène du mélange réactionnel obtenu et en le faisant recycler, caractérisé en ce que le gaz contenant de l'hydrogène à recycler est conduit, à une température située entre 450 et 650°C, sur un catalyseur contenant un métal du groupe constitué de nickel et de cobalt et que le gaz contenant de l-hydrogène est conduit sur un catalyseur à une vitesse spatiale de 0,25—20 litres (P.T.N.) par gramme de catalyseur par heure.